# EUROPÄISCHE PATENTSCHRIFT

(11) **EP 2 582 651 B1**
(45) Veröffentlichungstag und Bekanntmachung des Hinweises auf die Patenterteilung: **26.12.2018**
(21) Anmeldenummer: 11725111.6
(22) Anmeldetag: 16.06.2011
(51) Int. Cl.: C07C 45/50, C07C 47/02, C07C 5/333, C01B 3/26

(54) **ENERGIEEFFIZIENTE SYNTHESE VON ALIPHATISCHEN ALDEHYDEN AUS ALKANEN UND KOHLENDIOXID**
ENERGY-EFFICIENT SYNTHESIS OF ALIPHATIC ALDEHYDES MADE OF ALKANES AND CARBON DIOXIDE
SYNTHÈSE EFFICACE, SUR LE PLAN ÉNERGÉTIQUE, D'ALDÉHYDES ALIPHATIQUES À PARTIR D'ALCANES ET DE DIOXYDE DE CARBONE

(30) Priorität: 17.06.2010 DE 102010030209
(43) Veröffentlichungstag der Anmeldung: 24.04.2013
(73) Patentinhaber: Evonik Degussa GmbH, 45128 Essen (DE)
(72) Erfinder: FRANKE, Robert, 45772 Marl (DE); FRIDAG, Dirk, 45721 Haltern am See (DE); HAMERS, Bart, 46284 Dorsten (DE); ZANTHOFF, Horst-Werner, 45481 Mülheim a.d. Ruhr (DE); BLUG, Matthias, 58455 Witten (DE); STRAUTMANN, Julia, 91080 Marloffstein (DE); NORDHOFF, Stefan, 45657 Recklinghausen (DE)
(86) Internationale Anmeldenummer: PCT/EP2011/060021
(87) Internationale Veröffentlichungsnummer: WO 2011/157788

(56) Entgegenhaltungen:
- WO-A1-02/02496
- DE-A1-102004 041 850
- DE-A1-102005 061 642
- TOMINAGA K.: "An enviromentally friendly hydroformylation using carbon dioxide as a reactant catalysed by immobilized Ru-complex in ionic liquids", CATALYSIS TODAY, Bd. 115, 2006, Seiten 70-72, XP000002657704, in der Anmeldung erwähnt
- FUJITA S. ET AL.: "Hydroformylation of Cylcohexene with carbon dioxide and hydrogen using ruthenium carbonyl catalyst: influence of pressures of gasous components", INTERNATIONAL JOURNAL OF MOLECULAR SCIENCES, Bd. 8, 2007, Seiten 749-759, XP000002657705,
- SAKAKURA T. ET AL.: "Transformation of carbon dioxide", CHEMICAL REVIEWS, Bd. 107, 2007, Seiten 2365-2387, XP000002657706,
- ESSWEIN A.J. ET AL.: "Hydrogen production by molecular photocatalysis", CHEMICA REVIEWS, Bd. 107, 2007, Seiten 4022-4047, XP000002657707, in der Anmeldung erwähnt
- KUNG H H ET AL: "Oxidative dehydrogenation of alkanes over vanadium-magnesium-oxides", APPLIED CATALYSIS A: GENERAL, ELSEVIER, AMSTERDAM, NL, vol. 157, no. 1-2, 11 September 1997 (1997-09-11), pages 105-116, XP004338194, ISSN: 0926-860X, DOI: 10.1016/S0926-860X(97)00028-8

## Beschreibung

Aldehyde wie beispielsweise Valeraldehyd (Pentanal) sind vielfach verwendete Industriechemikalien. Pentanal wird heute großindustriell durch Hydroformylierung der Olefine 1-Buten oder 2-Buten unter Zugabe von Synthesegas (Kohlenmonoxid und Wasserstoff) dargestellt. Die Butene entstammen petrochemischen Prozessen, das Synthesegas wird ebenfalls aus fossilen Quellen hergestellt.

Angesichts der beschränkten wirtschaftlichen Verwertbarkeit der fossilen Rohstoffquellen und der als "klimaschädlich" angesehenen CO₂-Konzentration in der Atmosphäre besteht langfristig ein Bedarf an alternativen Wegen, Aldehyde wie Pentanal Ressourcen schonend und unter Einsparung von CO₂ herstellen zu können. Im Übrigen sieht sich das für die Hydroformylierung benötigte Kohlenmonoxid auf Grund seiner unbestrittenen Toxizität zunehmenden Akzeptanzproblemen in der Bevölkerung ausgesetzt.

Aufgabe der Erfindung ist es daher, ein Verfahren zur Herstellung von Aldehyden anzugeben, mit Hilfe dessen im Vergleich zur klassischen Hydroformylierung CO₂ eingespart werden kann, welches auf alternative Rohstoffquellen zurückgreift und welches auf die Bereitstellung von Kohlenmonoxid verzichten kann.

Gelöst wird diese Aufgabe durch Verfahren umfassend die folgenden Schritte:
a) Bereitstellung mindestens eines Alkans;
b) photokatalytische Dehydrierung des Alkans zu einem Gemisch enthaltend mindestens ein Olefin und Wasserstoff;
c) Zugabe von Kohlendioxid und Wasserstoff zu dem Gemisch;
d) Hydroformylierung des Olefins zu mindestens einem Aldehyd.

Die Hydroformylierung von Buten wird zum Beispiel in DE 10 2004 041 850 offenbart.

Eine erste Grundidee der vorliegenden Erfindung, die bei den Ansprüchen definiert ist, besteht darin, den bei der photokatalytische Dehydrierung von Alkanen freigesetzten Wasserstoff zur anschließenden Hydroformylierung des durch die Dehydrierung entstehenden Olefins zu verwenden. Der Wasserstoff-Bedarf der Hydroformylierung kann daher teilweise energieeffizient aus der CO₂-neutralen Photokatalyse gedeckt werden. Zudem begnügt sich die Photokatalyse mit Butanen als Ausgangsstoff, die im Vergleich zu den heute eingesetzten Butenen günstig verfügbar sind. Petrochemisch erhaltenes Butan wird heute unter CO₂-Emission überwiegend thermisch verwertet.

Zum Zweiten beruht die Erfindung auf der Erkenntnis, dass der aus der katalytischen Dehydrierung stammende Wasserstoff die Möglichkeit eröffnet, CO₂ anstelle von CO als Kohlenstoffquelle für die Hydroformylierung zu nutzen. Dies gelingt zum Beispiel im Wege einer Retro-Wassergas-Shift-Reaktion, mit Hilfe derer aus dem in der Atmosphäre unerwünschten CO₂ und dem freien Wasserstoff das für die Hydroformylierung benötigte CO in situ erzeugt werden kann. Eine separate Zuführung von giftigem CO entfällt, zudem kann in anderen Prozessen anfallendes CO₂ als Rohstoff genutzt werden, sodass die Kohlendioxid-Emission insgesamt sinkt.

Die Erfindung ermöglicht mithin die Rückführung von CO₂ in die chemische Wertschöpfungskette, indem das bisher benötigte Kohlenmonoxid, welches aus Synthesegas gewonnen wird, durch CO₂ ersetzt wird. Pro Tonne des produzierten Aldehyds würden, im Falle von Valeraldehyd, mehr als eine halbe Tonne CO₂ eingesetzt werden. Bei einer weltweiten Produktion von 340.000 t Valeraldehyd pro Jahr würde dies eine Rückführung von ca. 200.000 t CO₂ pro Jahr ermöglichen. Werden darüber hinaus auch andere Aldehyde durch dieses Verfahren hergestellt, so ist ein Vielfaches dieses Potentials zu erwarten.

Die beiden erfindungsgemäß kombinierten Verfahrensabschnitte "photokatalytische Dehydrierung" und "Hydroformylierung mit Kohlendioxid" sind für sich in der Literatur als machbar beschrieben:
So beschreiben K. Nomura und Y. Saito, in J. Chem. Soc., Chem. Commun, (1988), 161 die photokatalytische Dehydrierung von Alkanen unter milden Bedingungen und Strahlung an einem Rh-Katalysator:

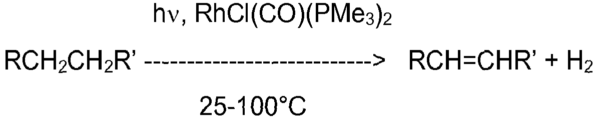

Weitere Handlungsanweisungen zur Durchführung von Photokatalyse geben:
a) A. J. Esswein, D. G. Nocera, Hydrogen Production by Molecular Photocatalysis, Chem. Rev. 2007, 107, 4022-4047,
b) D. Morales-Morales, R- Redón, C. Yung C. M Jensen, Dehydrogenation of alkanes catalyzed by an iridium phosphinito PCP pincer complex, Inorg. Chim. Acta, 2004, 357, 2953-2956;
c) M. J. Burk, R. H. Crabtree, D. V. McGrath, J. Chem. Soc., Chem. Commun. 1985, 1829-1830;
d) M. J. Burk, R. H. Crabtree, J. Am. Chem. Soc. 1987, 109, 8025-8032;
e) T. Sakakura, T. Sodeyama, Y. Tokunaga, M. Tanaka, Chem. Lett. 1988, 263-264 ;
f) K. Nomura, Y. Saito, J. Chem. Soc., Chem. Commun. 1988, 161;
g) J. A. Maguire, W. T. Boese, A. S. Goldman, J. Am. Chem. Soc. 1989, 111, 7088-7093;
K. Tominaga und Y. Sasaki offenbaren eine Hydroformylierung mit Kohlendioxid in Catal. Commun., 1, 2000, 1 wie folgt:

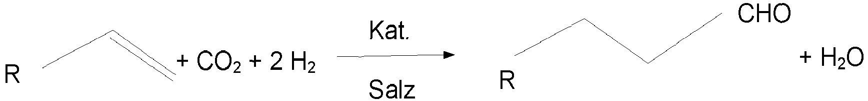

Als Katalysator für die Hydroformylierung mit CO₂ werden bevorzugt multinukleare Ruthenium-Komplexe wie bspw. Ru₃(CO)₁₂ eingesetzt.

Zusätzlich können Salze wie LiCI, LiBr, Lil, NaCl, KCl oder Mischungen daraus als Promoter zugegeben werden. Dies erscheint deshalb günstig, da in situ aus den Salzen gebildeten Halogen-Wasserstoff-Säuren (HCl, HBr ...) als Protonenüberträger wirken und so die Retro-Wassergas-Shift-Reaktion vermitteln.

Weitere brauchbare Handlungsanweisungen zur Durchführung der Hydroformylierung mit CO₂ enthalten:
a) K. Tominaga, Y. Sasaki, K. Hagihara, T. Watanabe, M. Saito, Chem. Lett. 1994, 1391-1394;
b) K. Tominaga, Y. Sasaki, J. Mol. Catal. A: Chem. 2004, 220, 159-165;
c) K. Tominaga, Catal. Today 2006, 115, 70-72;
d) S. Fujita, S. Okamura, Y. Akiyama, M. Arai, Int. J. Mol. Sci. 2007, 8, 749-759.

Bevorzugt wird das erfindungsgemäße Verfahren benutzt, um das Alkan n-Butan via photokatalytischer Dehydrierung zu dem Olefin 1-Buten und H₂ umzusetzen und dieses Gemisch unter Zugabe von CO₂ einer Hydroformylierung zu unterziehen, im Rahmen derer das 1-Buten in das Aldehyd Pentanal umgesetzt wird.

Der Gesamtprozess auf Basis von n-Butan und CO₂ umfasst die Basisschritte photokatalytische Dehydrierung und Hydroformylierung mit CO₂:

n-Butan → 1-Buten + H₂

(photokatalytische Dehydrierung)

1-Buten + CO₂ + 2 H₂ → Valeraldehyd + H₂O

(Hydroformylierung mit CO₂)

Valeraldehyd kann anschließend nach den bereits heute technisch etablierten Prozessen in großem Maßstab zur Herstellung von Industriechemikalien wie Polymeradditiven verwendet werden.

Für die Nutzung von CO₂ als Rohstoff wesentlich ist eine der Hydroformylierung vorgelagerte Umsetzung von Kohlendioxid und Wasserstoff in Wasser und Kohlenmonoxid, bei der es sich bevorzugt um eine Retro-Wassergas-Shift-Reaktion handelt.

Eine bevorzugte Weiterbildung der Erfindung sieht vor, die Umsetzung von Kohlendioxid und Wasserstoff in Wasser und Kohlenmonoxid und die Hydroformylierung in einem gemeinsamen Reaktor und/oder an einem gemeinsamen Katalysator ablaufen zu lassen.

Bevorzugt wird innerhalb der der photokatalytischen Dehydrierung und/oder bei der Hydroformylierung und/oder bei der Umsetzung der von Kohlendioxid und Wasserstoff in Wasser und Kohlenmonoxid ein immobilisierter Katalysator verwendet. Unter einem immobilisierten Katalysator ist ein homogenes Katalysatorsystem zu verstehen, welches an einem unlöslichen Trägerstoff gekoppelt ist, um den homogen wirkenden Katalysator wie einen heterogenen Katalysator aus der Reaktionsmischung abscheiden zu können. Die Immobilisierung gelingt mit Hilfe ionischer Flüssigkeiten:
Ionische Flüssigkeiten - nachfolgend IL genannt - sind niedrigschmelzende Salze (< 100 °C) und werden als neuartige Lösungsmittel z. B. für Extraktionsprozesse und die Katalyse eingesetzt. Ein wesentlicher Grund, warum ILs sowohl akademisches als auch industrielles Interesse geweckt haben, ist die Tatsache, dass sie einen extrem niedrigen Dampfdruck besitzen, wodurch Lösungsmittelverluste praktisch ausgeschlossen sind. Im Bereich des Einsatzes von ionischen Fluiden auf dem Gebiet der Katalyse werden in jüngster Zeit insbesondere zwei Ansatzpunkte verfolgt:

ILs sind nicht nur interessante Lösungsmittel für die homogene Katalyse, sondern auch für heterogene katalysierte Reaktionen. So konnten beispielsweise Xu et al.
(D.-Q. Xu, Z.-Y. Hu, W.-W. Li, S.-P. Luo, Z.-Y. Xu Hydrogenation in ionic liquids: An alternative methodology toward highly selective catalysis of halonitrobenzenes to corresponding haloanilines, J. of Mol. Cat. A: Chem. 2005, 235, 137 - 142.)
zeigen, dass ILs interessante Medien für die Hydrierung von halogenierten Nitrobenzolen zu den korrespondierenden halogenierten Anilinen an Raney-Nickel, kohlenstoffgeträgertem Pt- und Pd-Kontakten sind. Werden ILs als Lösungsmittel verwendet, wird die unerwünschte Dehalogenierung im Vergleich zu organischen Lösungsmitteln vermindert.

Auch das sogenannte SILP-Konzept (Supported Ionic Liquid Phase) ist vielversprechend. Dabei wird ein dünner Film einer IL mit einem in dieser gelösten homogenen Katalysator auf der Oberfläche eines porösen (inerten) Feststoffs z.B. durch Physisorption aufgebracht. Nachteil der homogenen Zweiphasenkatalyse werden dabei umgangen, da die benötigte IL-Menge reduziert wird und Stofftransporteffekte durch den dünnen Film vermieden werden. Für die Beschichtung des porösen Trägers wird die IL zusammen mit dem homogenen Katalysator in einem Lösungsmittel (z.B. Dichlorethan) gelöst und dann der feste Träger dazu gegeben. Die Lösung wurde dann langsam vom leichtflüchtigen Dichlormethan unter Vakuum befreit. Mit dieser Präparationsmethode kann sichergestellt werden, dass die IL vollständig in die Poren eindringt. Für die Herstellung von Systemen mit höherem oder niedrigerem Porenfüllgrad bzw. Schichtdicken kann die IL-Konzentration in der Dichlormethanlösung erhöht bzw. erniedrigt werden. Das SILP-Konzept wurde bereits für einige Reaktionen erfolgreich getestet.

Weitere Ausführungen zu SILP und deren Anwendung in der Katalyse entnimmt der Fachmann:
a) A. Riisager, P. Wasserscheid, R. v. Hal, R. Fehrmann, Continous fixed-bed gas-phase hydroformylation using supported ionic liquid phase (SILP) Rh catalysts, J. Catal. 2003, 219, 452-455;
b) Riisager, K. M. Eriksen, P. Wasserscheid and R. Fehrmann, Propene and 1-Octene Hydroformylation with Silica-Supported, Ionic Liquid-Phase (SILP) Rh-Phosphine Catalysts in Continuous Fixed-Bed Mode, Catal. Letters 2003, 90, 149-153;
c) C. P. Mehnert, R. A. Cook, N. C. Dispenziere, M. Afeworki, Supported Ionic Liquid Catalysis - A New Concept for Homogeneous Hydroformylation Catalysis, J. Am. Chem. Soc. 1998, 120, 12289-12296;
d) C. P. Mehnert, E. J. Molzeleski, R. A. Cook, Supported ionic liquid catalysis investigated for hydrogenation reactions, Chem. Comm. 2002, 24, 3010-3011;
e) A. Wolfson, I. F. J. Vankelecom, P. A. Jacobs, Co-immobilization of transition-metal complexes and ionic liquids in a polymeric support for liquid-phase hydrogenations, Tetrahedron Lett. 2003, 44, 1195-1198;
f) C. M. Gordon, New developments in catalysis using ionic liquids, Appl. Catal. A: General 2001, 222, 101-117;
g) P. J. Dyson, Transition metal chemistry in ionic liquids, Trans. Met. Chem. 2002, 27, 353-358;
h) D. Zhao, M. Wu, Y. Kou, E. Min, lonic liquids: applications in catalysis, Cat. Today 2002, 74, 157-189;
i) J. Dupont, R. F. de Souza, P. A. Z. Suarez, lonic Liquid (Molten Salt) Phase Organometallic Catalysis, Chem. Rev. 2002, 102, 3667-3692;
j) H. Olivier-Bourbigou in Catalysis in Nonaqueous lonic Liquids in Multiphase Homogeneous Catalysis (B. Cornils et al. Eds.), Wiley VCH, Weinheim, Germany, 2006, 407-603;
k) M. J. Earle, P. B. McCormac, K. R. Seddon, The first high yield green route to a pharmaceutical in a room temperature ionic liquid, Green Chem. 2000, 2, 261;
l) B. Hamers, P. S. Bäuerlein, C. Müller, D. Vogt, Hydroaminomethylation of n-Alkenes in a Biphasic lonic Liquid System, Adv. Synth. Catal. 2008, 350, 332-342;
m) H. Wong, S. Han, A.G. Livingston, The effect of ionic liquids on product yield and catalyst stability, Chem. Eng. Sci. 2006, 61, 1338-1341.
n) K. Anderson, P. Goodrich, C. Hardacre. D.W. Rooney, Heterogeneously catalysed selective hydrogenation reactions in ionic liquids, Green Chem. 2003, 5, 448 - 453. k) D.-Q. Xu, Z.-Y. Hu, W.-W. Li, S.-P. Luo, Z.-Y. Xu Hydrogenation in ionic liquids: An alternative methodology toward highly selective catalysis of halonitrobenzenes to corresponding haloanilines, J. of Mol. Cat. A: Chem. 2005, 235, 137 - 142.

Ein weiteres Konzept ist das sogenannte SCILL-Konzept (Solid Catalyst with lonic Liquid Layer) zur Verbesserung der Selektivität heterogener Katalysatoren. Das SCILL-Konzept -vgl.
a) U. Kernchen, B. Etzold, W. Korth, A. Jess, Solid Catalyst with lonic Liquid Layer (SCILL) - A New Concept to Improve the Selectivity Investigated for the Example of Hydrogenation of Cyclooctadiene. Chem. Eng. Technol. 2007, 30, 985-994.
b) N. Wörz, J. Arras, P. Claus, Einfluss ionischer Flüssigkeiten auf die kontinuierliche Hydrierung von Citral im Rieselbettreaktor. Jahrestreffen Reaktionstechnik, Würzburg, 10. - 12.5.2010, Tagungsband, S. 34/35 -
kombiniert und erweitert in gewisser Weise die beiden zuvor beschriebenen Strategien: Ähnlich wie bei der SILP-Technologie wird ein poröser Feststoff mit einer ionischen Flüssigkeit beschichtet, allerdings ist der Feststoff jetzt ein heterogener Katalysator und nicht nur ein inerter Träger, der den in der IL gelösten homogenen Katalysator immobilisieren soll. Ein homogener Katalysator ist somit beim SCILL-Konzept nicht beteiligt, wenngleich eine Kombination mit dem SILP-Konzept eine Option für eine Integration von homogener und heterogener Katalyse darstellt. Die IL-Schicht ist stabil und ein Auswaschen der IL in die organische Phase tritt nicht auf.

Neben dem Vorteil der Möglichkeit der Immobilisierung eines homogenen Katalysators durch die IL auf einem porösen Träger kann die ionische Flüssigkeit auch einen direkten Einfluss auf die Kinetik der Reaktion haben. Dies gilt insbesondere dann, wenn die Löslichkeit der Reaktanden bzw. Zwischenprodukte unterschiedlich ist. So wird z. B. bei der sequentiellen heterogen-katalysierten Hydrierung von Cyclooctadien (COD) in Cycloocten (COE) und Cyclooctan (COA) durch die Beschichtung mit einer ionischen Flüssigkeit (Butylmethylimidazolium-octylsulfat) die Selektivität zum Zwischenprodukt COE deutlich gesteigert. Dies liegt unter anderem an der schlechten Löslichkeit des Zwischenproduktes. Ein ähnlicher Effekt ist auch für die erfindungsgemäß wichtige Retro-Wassergas-Shift-Reaktion (H₂ + CO₂ → CO + H₂O) zu erwarten, da CO₂ von allen vier beteiligten Reaktionspartnern die beste Löslichkeit in ILs haben dürfte, was einen positiven Einfluss auf diese bei relativ tiefen Temperaturen gleichgewichtslimitierte Reaktion haben wird.

Das grundlegende Problem der homogenen Katalyse, die Abtrennung des Katalysators von der Reaktionsmasse, wird also erfindungsgemäß durch eine Immobilisierung der entwickelten homogenen Katalysatoren mit Hilfe ionischer Fluide durch das oben beschriebene SILP und/oder SCILL-Konzept gelöst.

Dieses Konzept kann dabei auch auf die photokatalytische Umsetzung von Alkanen übertragen werden. Als Trägermaterial können poröse Gläser eingesetzt werden, um eine möglichst hohe Eindringtiefe des Lichtes sicherzustellen. Der für die photokatalytische Dehydrierung der Alkane verwendete Katalysator wird also bevorzugt von einem porösen Glas getragen.

### Beispiel

Am Beispiel der Herstellung von Valeraldehyd (Pentanal) aus n-Butan soll die Erfindung nun näher erläutert werden:
Erfindungsgemäß wird n-Butan zunächst photokatalytisch dehydriert und das erhaltene 1-Buten mit CO₂ in einer Hydroformylierung zum Valeraldehyd umgesetzt.

Dieser Gesamtprozess sieht wie folgt aus:

| | |
|---|---|
| 1) | n-Butan → 1-Buten + H₂ (photokatalytische Dehydrierung) |
| 2a) | H₂ + CO₂ → CO + H₂O |
| 2b) | 1-Buten + CO + H₂ → Valeraldehyd |
| ∑: | n-Butan + CO₂ → Valeraldehyd + ½ O₂ |

Die Schritte 2a und 2b können in einem Reaktor bzw. möglichst an einem Katalysator ablaufen und können daher wie folgt als CO₂-Hydroformylierung aufgefasst werden:

2a/b) 1-Buten + CO₂ + 2 H₂ → Valeraldehyd + H₂O

Auch ohne eine photokatalytische Butandehydrierung, d.h. auf der Basis von Buten wäre der Prozess im Vergleich zur "üblichen" CO-basierten Hydroformylierung immer noch vorteilhaft, sofern der Wasserstoff CO₂-neutral bereitgestellt werden kann:

| | |
|---|---|
| 1) | 2 H₂O → 2 H₂ + O₂ (möglichst CO₂-neutral; hier nicht näher beschrieben) |
| 2a) | H₂ + CO₂ → CO + H₂O |
| 2b) | 1-Buten + CO + H₂ → Valeraldehyd |
| ∑: | 1-Buten + CO₂ + H₂O → Valeraldehyd + O₂ |

Für die Photokatalyse geeignete Katalysatoren sind organische und metallorganische Photosensibilisatoren auf Basis von Iridium und Rhodium bzw. Kombinations-Katalysatoren, bestehend aus einer Photosensibilisatorkomponente und einer Protonenreduktionskomponente auf Basis von Pd, Ru, Ir oder Fe.

Für die Hydroformylierung geeignete Katalysatoren sind Iridium-Phosphin-Komplexe, Ruthenium-basierte Komplexe, insbesondere multinukleare Ruthenium-Komplexe sowie Eisenhydridcarbonyl-Komplexe. Als Promoter können zusätzlich Salze wie LiCI, LiBr, Lil, NaCl, KCl allein oder in Mischungen daraus zugegeben werden.

Die homogenen Katalysatorsysteme können durch ionische Flüssigkeiten auf festen porösen Trägern unterschiedlicher Porengröße immobilisiert werden. Zur Erhöhung der Lichtdurchlässigkeit können als Träger poröse Gläser verwendet werden.

## Patentansprüche

1. Verfahren zur Herstellung von Aldehyden aus Alkanen,
**gekennzeichnet durch** die folgenden Schritte:
a) Bereitstellung mindestens eines Alkans;
b) photokatalytische Dehydrierung des Alkans zu einem Gemisch enthaltend mindestens ein Olefin und Wasserstoff;
c) Zugabe von Kohlendioxid und Wasserstoff zu dem Gemisch;
d) Hydroformylierung des Olefins zu mindestens einem Aldehyd,
wobei eine der Hydroformylierung vorgelagerte Umsetzung von Kohlendioxid und Wasserstoff in Wasser und Kohlenmonoxid erfolgt und es sich bei der Umsetzung von Kohlendioxid und Wasserstoff in Wasser und Kohlenmonoxid um eine Retro-Wassergas-Shift-Reaktion handelt.

2. Verfahren nach Anspruch 1,
**dadurch gekennzeichnet,**
• **dass** es sich bei dem Alkan um n-Butan handelt,
• **dass** es sich bei dem Olefin um 1-Buten handelt,
• und **dass** es sich bei dem Aldehyd um Valeraldehyd handelt.

3. Verfahren nach Anspruch 1 oder 2,
**dadurch gekennzeichnet,**
**dass** die Umsetzung von Kohlendioxid und Wasserstoff in Wasser und Kohlenmonoxid und die Hydroformylierung in einem gemeinsamen Reaktor und/oder an einem gemeinsamen Katalysator ablaufen.

4. Verfahren nach einem der vorhergehenden Ansprüche, **gekennzeichnet durch** die Verwendung eines immobilisierten Katalysators bei der photokatalytische Dehydrierung und/oder bei der Hydroformylierung und/oder bei der Umsetzung der von Kohlendioxid und Wasserstoff in Wasser und Kohlenmonoxid.

5. Verfahren nach Anspruch 4,
**dadurch gekennzeichnet,**
**dass** der Katalysator mittels einer ionischen Flüssigkeit immobilisiert ist.

6. Verfahren nach Anspruch 5,
**dadurch gekennzeichnet,**
**dass** der für photokatalytische Dehydrierung der Alkane verwendete Katalysator von einem porösen Glas getragen wird.

7. Verfahren nach einem der vorhergehenden Ansprüche,
**dadurch gekennzeichnet,**
**dass** als Katalysator für die Hydroformylierung multinukleare Ruthenium-Komplexe, insbesondere Ru₃(CO)₁₂ eingesetzt werden.

8. Verfahren nach einem der vorhergehenden Ansprüche,
**dadurch gekennzeichnet,**
**dass** der Hydroformylierung mindestens ein Salz, insbesondere ausgewählt aus der Gruppe umfassend LiCl, LiBr, LiI, NaCl, KCl zugegeben wird.

## Claims

1. A process for producing aldehydes from alkanes, **characterized by** the following steps:
a) providing at least one alkane;
b) photocatalytically dehydrogenating the alkane to a mixture comprising at least one olefin and hydrogen;
c) adding carbon dioxide and hydrogen to the mixture;
d) hydroformylating the olefin to at least one aldehyde,
a conversion of carbon dioxide and hydrogen into water and carbon monoxide upstream of the hydroformylating taking place and the conversion of carbon dioxide and hydrogen into water and carbon monoxide being a reverse water gas shift reaction.

2. A process according to claim 1,
**characterized in that**
• the alkane is n-butane,
• the olefin is 1-butene, and
• the aldehyde is valeraldehyde.

3. A process according to claim 1 or 2,
**characterized in that**
the conversion of carbon dioxide and hydrogen into water and carbon monoxide and the hydroformylating take place in a conjoint reactor and/or over a conjoint catalyst.

4. A process according to any preceding claim, **characterized by** the use of an immobilised catalyst in the photocatalytic dehydrogenating and/or in the hydroformylating and/or in the conversion of carbon dioxide and hydrogen into water and carbon monoxide.

5. A process according to claim 4,
**characterized in that**
the catalyst is immobilised using an ionic liquid.

6. A process according to claim 5,
**characterized in that**
the catalyst used for photocatalytically dehydrogenating the alkanes is supported by a porous glass.

7. A process according to any preceding claim,
**characterized in that**
the catalyst for the hydroformylating is a multinuclear ruthenium complex, more particularly Ru₃(CO)₁₂.

8. A process according to any preceding claim,
**characterized in that**
at least one salt is added to the hydroformylating step, more particularly a salt selected from the group comprising LiCl, LiBr, LiI, NaCl, KCl.

## Revendications

1. Procédé de fabrication d'aldéhydes à partir d'alcanes, **caractérisé par** les étapes suivantes :
a) la préparation d'au moins un alcane ;
b) la déshydrogénation photocatalytique de l'alcane en un mélange contenant au moins une oléfine et de l'hydrogène ;
c) l'ajout de dioxyde de carbone et d'hydrogène au mélange ;
d) l'hydroformylation de l'oléfine en au moins un aldéhyde,
une transformation du dioxyde de carbone et de l'hydrogène en eau et monoxyde de carbone ayant lien en amont de l'hydroformylation, et la transformation du dioxyde de carbone et de l'hydrogène en eau et monoxyde de carbone étant une réaction du gaz à l'eau inverse.

2. Procédé selon la revendication 1, **caractérisé en ce que**
- l'alcane est le n-butane,
- l'oléfine est le 1-butène,
- et l'aldéhyde est le valéraldéhyde.

3. Procédé selon la revendication 1 ou 2, **caractérisé en ce que** la transformation du dioxyde de carbone et de l'hydrogène en eau et monoxyde de carbone et l'hydroformylation se déroulent dans un réacteur commun et/ou sur un catalyseur commun.

4. Procédé selon l'une quelconque des revendications précédentes, **caractérisé par** l'utilisation d'un catalyseur immobilisé lors de la déshydrogénation photocatalytique et/ou lors de l'hydroformylation et/ou lors de la transformation du dioxyde de carbone et de l'hydrogène en eau et monoxyde de carbone.

5. Procédé selon la revendication 4, **caractérisé en ce que** le catalyseur est immobilisé au moyen d'un liquide ionique.

6. Procédé selon la revendication 5, **caractérisé en ce que** le catalyseur utilisé pour la déshydrogénation photocatalytique des alcanes est supporté par un verre poreux.

7. Procédé selon l'une quelconque des revendications précédentes, **caractérisé en ce que** des complexes de ruthénium multinucléaires, notamment Ru₃(CO)₁₂, sont utilisés en tant que catalyseur pour l'hydroformylation.

8. Procédé selon l'une quelconque des revendications précédentes, **caractérisé en ce qu'**au moins un sel, notamment choisi dans le groupe comprenant LiCl, LiBr, LiI, NaCl, KCl, est ajouté à l'hydroformylation.
